# EUROPEAN PATENT SPECIFICATION

(11) **EP 3 439 728 B1**
(45) Date of publication and mention of the grant of the patent: **19.02.2025**
(21) Application number: 17778793.4
(22) Date of filing: 04.04.2017
(51) Int. Cl.: A61M 31/00, A61M 25/10

(54) **DRUG DELIVERY APPARATUS**
VORRICHTUNG ZUR WIRKSTOFFFREISETZUNG
APPAREIL D'ADMINISTRATION DE MÉDICAMENT

(30) Priority: 04.04.2016 US 201662317657 P
(43) Date of publication of application: 13.02.2019
(73) Proprietor: Innoventions Ltd., 30600 Or Akiva (IL)
(72) Inventor: YACHIA, Daniel, 46762 Herzliya Pituach (IL); SHTIGLITZ, Ortal, 6603254 Tel Aviv (IL); ARBEL, Yaara, 1795000 Kibbutz Bet Rimon (IL)
(74) Representative: Arth, Hans-Lothar
(86) International application number: PCT/IL2017/050409
(87) International publication number: WO 2017/175222

(56) References cited:
- CN-A- 103 656 836
- US-A- 4 364 392
- US-A- 5 993 473
- US-A1- 2002 165 521
- US-A1- 2003 195 456
- US-A1- 2004 087 902
- US-A1- 2006 155 163
- US-A1- 2010 016 834
- US-A1- 2010 274 222
- US-A1- 2013 116 655

## Description

### FIELD OF THE INVENTION

The present invention is directed to drug delivery devices in body cavities, and in particular for drug delivery in the bladder.

### BACKGROUND OF THE INVENTION

The following prior art publications are considered to be relevant for an understanding of the background of the invention:
De Bruijn E. A. et al., Pharmacodynamics and H. P. pharmacokinetics of intravesical mitomycin C upon different dwelling times, Int. J. Cancer. 1992; 51:359-364.
Song D.,. Wientjes M. G, Au J. L., Bladder tissue pharmacokinetics of intravesical taxol, Cancer Chemother. Pharmacol. 1997; 40:285-292.
Schenk-Braat E.A., Bangma C.H., Immunotherapy for superficial bladder cancer, Cancer Immunol. Immunother. 2005; 54: 414-423.
Shen Z, Shen T, Wientjes MG, O'Donnell MA, Au JL, Intravesical Treatments of Bladder Cancer: Review, Pharm Res. 2008; 25: 1500-1510.
Lammers, R. J. M. et al., The role of a combined regimen with intravesical chemotherapy and hyperthermia in the management of non-muscle-invasive bladder cancer: a systematic review. Eur. Urol. 2011;60, 81-93.

US Patent Application Publication No. US 2010/0016834 A1

US 5,993,473 describes an expandable device with a fillant delivery tube 18' and an expandable body 16'. The expandable body may be slightly taut while in a non-expanded configuration where the tip of the tube contacts the interior surface of the expandable body.

The field of targeted, site-specific, controlled drug delivery is developing rapidly. The primary advantage of this targeting is the delivery of drugs directly at the diseased site, which allows for high local doses to be administered without exceeding the systemic toxicity level of the drug, as well as providing effective drug doses by continuous and constant drug delivery lasting from several days to several months and, maintaining therapeutic levels of the drug at the target site. The exact targeted release ensures efficiency and allows controlled release of mono- and combination therapies. This is especially important for drugs, which are usually administered orally orintravenously, which after losing to systemic metabolism, a large portion of the administered drug fails to reach their target, or reaches the target in low therapeutic concentrations. Additionally, these orally or intravenouslyadministered drugs typically result in various systemic side effects.

One site, which is showing promise for intravesical treatments, is the bladder. One reason for focusing on the bladder is that it is easily accessed through the urethra. Contemporary bladder intravesical treatments involve repeated bladder instillations of a diluted drug which can be kept in the bladder for about 2 hours and then drained from the bladder.

Drug transport in bladder tissues, is a function of time and its distance from the bladder cavity. For drug transport purposes, the bladder wall is divided into two sections, the urothelium (mucosa) that is not blood-perfused, and the submucosal and muscle layers of the bladder that contain blood vessels and lymphatics. Transport of a drug from the urine in the bladder across the urothelium which is about 200 µm thick and made from about 7-10 cell layers occurs by diffusion across this single homogeneous barrier. The second barrier for drug transport is the submucosa and superficial muscle which has a porous structure, 200-4,000 µm thick and the drug is absorbed through its blood vessels. The drug concentration in this layer declines by about 50%, depending on the amount of blood vessels which increase in deeper tissue layers. Another determinant in drug penetration through the urothelium is the size of the drug molecule and its lipophilicity, the smaller the lipophilic drug molecule, the higher its systemic absorption.

### SUMMARY OF THE INVENTION

Throughout this document, the term "drug" includes drugs, chemo- or immunotherapeutic agents, medicines, natural compositions, pharmaceutical compositions, nutroceutical compositions, or any substance such as cells or viruses used to treat or otherwise cause an effect in the mammalian body.

The invention is defined in independent claim 1. Further aspects of the invention are defined in dependent claims 2-9.

The present invention, in some embodiments, is directed to a device for drug delivery into the bladder. The urinary bladder is easily accessed through the urethra. Research has found that the bladder inner lining, the urothelium, limits therate of absorption of molecules into the systemic circulation. For most small-molecule drugs, less than 5% of the dose is absorbed into the systemic circulation which is important especially with drugs related to cancer (De Bruijn E. A. et al., Song D. et al). Based in these characteristics, the urothelium is showing promise in retaining pharmacologically active agents for extended periods of time. By delivering drugs directly to the urothelium, and limiting the systemic absorption of certain therapeutic agents, the therapeutic agents can be delivered in effective concentrations. The rationale for intravesical therapy is to maximize the exposure of the drugs to attack the diseases at the level of the urothelium or immediately beneath it, and prevent or reduce systemic dispersion of the drugs.

### Urge Incontinence (UI)/ Overactive Bladder (OAB)

Embodiments of the present invention are also directed to drug delivery directly to the urothelium to treat urge urinary incontinence (UI)due to overactive bladder. This is the most common type of UI in the elderly population. Urge incontinence is characterized by abrupt urgency, frequency and nocturia not related to the anatomical capacity of the bladder or deficient sphincters. The volume of leakage may be small or large. The prevalence of UI is difficult to measure, due to under-reporting. However, studies conducted in the US and other parts of the world appear to show that a significant number of people suffer from the disorder.

The findings of a large survey conducted by the Sifo/Gallop network across 6 European countries (UK, France, Germany, Italy, Spain and Sweden), investigating the prevalence of OAB indicated that the number of people over 40 years with OAB symptoms in these countries would be over 22 million. According the study, the overall prevalence of UI was 16.6% in people aged over 40. Prevalence increased with increasing age, and ranged from 12% (France) to 20% (Spain). Frequency was the most common reported symptom (85%), followed by urgency.

A study performed at the Norwegian University of Science and Technology in Trondheim found that 29% of their respondents reported incontinence.

### Interstitial Cystitis / Painful Bladder Syndrome

Interstitial cystitis (IC), which is often called "painful bladder", is a chronic bladder problem. It most often affects women and can have a long-lasting impact on the quality of life. It causes a chronic pelvic pain and in some patients the bladder pressure may cause the patient to empty his or herbladder over tentimes a day to relieve their pain and pressure. In women, this pain expands to the genitals and causes pain during sexual intercourse. In men, pain can expand to the external genital organs, and the perineum, and also may cause painful orgasms or pains after sex in the genital organs. There is alsoa burning feeling during urination. All these patients have a bladder inflammation and up to 10% may have ulcerations in their bladder. The severity of the IC symptoms often varies and sometimes disappears. Although the symptoms of IC resemble the symptoms of a bladder infection, the urine of these patients does not contain any bacteria. The cause of IC is not known, but many factors, such as a defect in the bladder urothelium causing toxic substances in the urine to leak into the bladder tissues and causing the pain and irritation, an autoimmune reaction, allergy, infection or heredity, are thought to be involved.

There are no standard treatment protocols for IC. Treatments for ICincludeoral medications(amitryptiline), intravesical instillations (DMSO, heparin, lidocaine),fulgurations, hydrodistension, neuromodulation, and even cystectomy (removal of the bladder) and urinary diversion .

### Superficial Bladder Cancer and Carcinoma-in-situ

Embodiments of the present invention are also directed to drug delivery directly to the urothelium to treat bladder cancer, and in particular, superficial bladder cancer. Bladder cancer is the fifth most common cancer in the United States, with an estimated 67,160 newly diagnosed cases and 13,750 deaths in the United States in 2007. The standard treatment for patients with superficial bladder cancer is surgical transurethral resection (TUR) of tumors, with an 80% early success rate. However, nearly 70% of these patients will develop tumor recurrence, with 25% showing progression to muscle-invading disease, within 5 years after TUR (Schenk-Braat E.A., and Bangma C.H.).

Worldwide there are 500,000 patients per year with superficial bladder cancer. This takes into account the fact that 70% are recurrent patients from the previous year. Intravesical chemotherapy, clinical hyperthermia and immunotherapy are widely used as adjuvant therapies after TUR, to prevent recurrence and progression of superficial disease. (Shen Z, Shen T, Wientjes MG, O'Donnell MA, and Au JL). Thermotherapy alone or combined with chemotherapy or radiotherapy are additional options. In clinical hyperthermia certain body tissues are exposed to temperatures of 43.5-44°C to damage or kill cancer cells or make them more sensitive to certain chemotherapeutic drugs and irradiation..Clinical hyperthermia combined with intravesical chemotherapyor radiotherapy isknown to enhance the effects of thermotherapy(Lammers, R. J. M. et al.).

The present invention includes the delivery to the urothelium of intravesical chemotherapeutic agents for reduction of superficial bladder tumor reoccurrence. These chemotherapeutic agents may beused in addition to TUR, and include, for example, mitomycin C (MMC), doxorubicin, epirubicin, thiotepa, ethoglucid, valrubicin, cisplatin, gemcitabine, suramin or their combinations.Adding an externally controlled and/or activated heating element to the drug delivery balloon for clinical level hyperthermia can enhance the efficacy of the chemotherapy.

In carcinoma in situ (CiS), cancer cells are only in the lining of the bladder. CiS can occur in patches throughout the bladder lining and the cells are very abnormal. CiS has a high risk of spreading into the deeper layers of the bladder.

The present invention includes the delivery to the urothelium of immunomodulating Agents such as Bacille Calmette-Guérin (BCG) and immunoregulators such as interferons (IFN), interleukin-2 (IL-2), interleukin-12 (IL-12), tumor necrosis factor (TNF), keyhole limpet hemocyanin (KLH) and rubratin have activity in BCG-refractory patients. They are used either for the treatment of carcinoma in situ (CiS) of the bladder or after TUR for prophylaxis of superficial bladder cancer.

### Chronic Bladder Infections

Embodiments of the present invention are also directed to drug delivery directly to the urothelium to treat chronic bladder infections. By delivering directly to the urothelium, the present invention provides antibiotics, such as low-cost antibiotics, antibiotics which are too toxic for systemic use, and an antiseptics.

Embodiments of the present invention are directed to intravesical devices, comprising a balloon made of silicone or other elastic and permeable materials. The devices may float on the urine, or sink in the urine. The permeability of the balloon material can be modified either during its production or by drilling micro-holes of predetermined sizes and numbers. Floating of the balloon in the bladder tends to allow prolongation the residence time of the drug much more than the presently known bladder instillations of 2-3 hours, and releases either water and/or oil soluble drugs or agents through its walls atconstant and predetermined rates and for predetermined time periods. The device can remain in the bladder for up to 90 days or more. The balloon is either removed or replaced with a new one when the drug is depleted.

The devices of the present invention are used to deliver chemo- or immunotherapeutic agents, anti-cholinergics, non-steroidal anti-inflammatory drugs, antibiotics alone or in combinations, antiseptics to the bladder, specifically the urothelium. By the aforementioned treatment, more powerful single drugs or combinations of drugs can be administered for treatment, symptom management and improvement of the quality of life of patients. Drugs which are water or oil soluble can be combined and released from the same balloon. Combinations of different oils, air or gasses with oil/s can be used for allowing floatability with the oil used also as a matrix for dissolving and also for carrying therapeutic agents.

The devices are insertable and removable non-surgically, for example, in an outpatient or clinical setting. The insertion and removal of the device can be done by physicians, nurses and other trained medical personnel. The device insertion and retrieval instruments include, for example those described in US Patent Application Publication No. US 2010/0016834 A1. These devices are advantageous as they do not require any endoscopic manipulation in their operation. Another alternative is the use of an endoscope for the insertion or retrieval of the device.

Thus, the present invention provides a medical device for insertion into a body cavity comprising:
- a core;
- an expandable balloon surrounding the core, the balloon having a wall made from an elastic permeable material, the balloon having an empty configuration and an inflated configuration, the balloon wall being stretched in the empty configuration and applied to a surface of the core, and the balloon having a lumen in the inflated configuration; and
- a valve configured to allow introduction of one or more fluids between the wall of the balloon and the surface of the core.

In the device of the invention, the core may be made from a rigid or semi-rigid material. The core may be hollow.

The wall of the balloon may form a fluid impervious seal with the core. The device may further comprise one or more magnetic elements. The magnetic elements may be embedded in the wall of the balloon.

In the device of the invention, the balloon may further comprise one or more additional lumens.

The device of the invention may be adapted for insertion into a urinary bladder.

In another of its aspects, the invention provides a system comprising:
(a) an implantable medical device according to claim 1.
(b) one or more instruments selected from an insertion instrument for inserting the device into the body cavity and a retrieval instrument for retrieving the device from the body cavity.

Aso provided is a method (not part of the claimed invention) for treating a body cavity comprising:
(a) providing an implantable medical device comprising:
   - a core;
   - an expandable balloon surrounding the core, the balloon having a wall made from an elastic permeable material, the balloon having an empty configuration and an inflated configuration, the balloon wall being stretched in the empty configuration and applied to a surface of the core, and the balloon having a lumen in the inflated configuration; and
   - a valve configured to allow introduction of one or more fluids between the wall of the balloon and the surface of the core;
(b) inserting the device into the body cavity; and,
(c) inflating the balloon with at least one solution or suspension by injecting the solution or suspension through the valve between the surface of the core and the wall of the balloon.

Unless otherwise defined herein, all technical and/or scientific terms used herein have the same meaning as commonly understood by one of ordinary skill in the art to which the invention pertains. Although methods and materials similar or equivalent to those described herein may be used in the practice or testing of embodiments of the invention, exemplary methods and/or materials are described below. In case of conflict, the patent specification, including definitions, will control. In addition, the materials, methods, and examples are illustrative only and are not intended to be necessarily limiting. The methods herein are not claimed.

### BRIEF DESCRIPTION OF THE DRAWINGS

Some embodiments of the present invention are herein described, by way of example only, with reference to the accompanying drawings. With specific reference to the drawings in detail, it is stressed that the particulars shown are by way of example and for purposes of illustrative discussion of embodiments of the invention.

Attention is now directed to the drawings, where like reference numerals or characters indicate corresponding or like components. In the drawings:
**FIG. 1A** shows a cross-sectional view of a device for treating a body cavity having a balloon formed from an elastic material, the balloon being in an empty state, in accordance with embodiments of the present invention;
**FIG. 1B** shows a cross-sectional view of the device of Fig. 1A with the balloon in an inflated state;
**FIG. 1C** shows a cross-sectional view of a device for treating a body cavity having a balloon with the balloon having a dimpled surface in the inflated state;
**FIG. 2** shows a cross-sectional view of the device of Fig. 1 inflated with oil based solution or suspension and an aqueous based solution or suspension;
**FIG. 3** A shows a cross-sectional view of a balloon-shaped device with a hollow core in accordance with embodiments of the present invention;
**FIG. 3B** shows a cross-sectional view of the device of FIG. 3A including ferromagnetic particles in the wall of the balloon, in accordance with embodiments of the present invention;
**FIG. 3C** shows a cross-sectional view of the device of FIG. 3A including ferromagnetic particles in the lumen of the device, in accordance with embodiments of the present invention;
**FIG. 4A** is a cross-sectional view of device having two balloons, one inside the other, in a non-inflated state, in accordance with embodiments of the present invention; and
**FIG. 4B** shows the device of Fig. 4A after inflating the two balloons.

### DESCRIPTION OF THE INVENTION

In one of its aspects, the present invention provides a device for delivery into a body cavity, such as the urinary bladder, of such compounds as drugs, therapeutic agents, neutriceuticals, collectively referred to herein as *"drugs"*

The device includes an inflatable balloon that serves as a reservoir for a solution or suspension containing a drug for controlled sustained release. The wall of the balloon is made from an elastic permeable material that allows diffusion of molecules through the wall of the balloon.

The device may include a balloon-in-balloon configuration where the balloons can be inflatable alone or in combination. In this case, the wall of the outer balloon is made from an elastic material that allows diffusion of molecules through the wall of the outer balloon and serves as a reservoir for holding a drug for controlled sustained release, by diffusing through the walls of the outer balloon. The inner balloon is designed to be inflated with a substance to impart to the system a desired specific gravity. Thus, for example, the inner balloon may be filled with air, gas or oil for buoyancy of the system in urine.

After inflating the balloon with a solution or suspension containing the substances and inserting the device into the body cavity, the substances are released by diffusion through the wall of the system into the cavity.

The device is inserted into the body cavity and may be subsequently removed from the body cavity. In the case of the urinary bladder, the device can be inserted and removed from the bladder, via the urethra, in both males and females. The device is preferably delivered into the body cavity in an empty state. After delivery of the device to the body cavity, the balloon is inflated with a solution or suspension containing the substance or substances to be released into the urine. The substances to be released may be dissolved or suspended, for example, in water or oil. Oils that can be used include, for example, silicon oils, vegetable oils or mineral oils such as Superla Light mineral oil from Chevron Corp. The inflated balloon may be left in the body cavity for long periods of time. The device can be removed when the treatment cycle is completed or the drug reservoir/s is depleted, and if necessary, replaced with a new device. The balloon can be inflated with numerous drugs to be delivered to the cavity separately or in combination, for example, in effective amounts, for the treatment of various disorders, such as interstitial cystitis, overactive bladder/urge incontinence, superficial bladder tumors and chronic bladder infections.

The device of the invention may be deployed in the body cavity in a single insertion. After its insertion, by loading sustained and other controlled release drug solutions, the balloon can remain in the cavity for long time periods, making it suitable for long term treatments, instead of, for example, the contemporary trans-urethral instillations necessitating repeated catheterizations, which heighten the risks of urethral injuries and urinary tract infections.

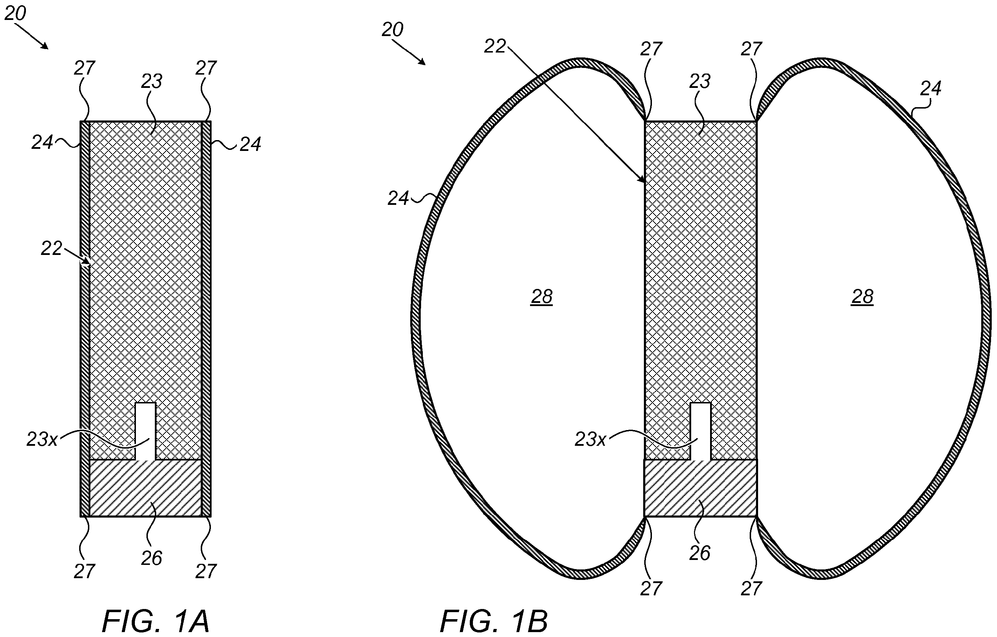

FIGS. 1A, and 1B show a device 20, for drug delivery in accordance with one embodiment of the present invention. The device 20 is shown in FIG. 1A in its empty configuration of the device and in Fig. 1b in an inflated configuration.

The device 20 includes an inner core 22 formed from a rigid material and an elastic skin or wall 24 to be inflated to form a balloon. The wall 24 attaches to the core 22, around the edges 27of the core 22, the attachment being such that a fluid impervious seal, against fluids, such as air, liquid, oil, and the like, is formed. The balloon 24 is made from an elastic material that allows diffusion though the wall of the balloon of the substances to be released. One suitable material for the wall 24 is a type of silicone material known commercially as ELASTOSIL^{®} LR 3003 from Wacker Chemie AG. This material is also punctureable by standard medical needles, such that the devices 20 can be deflated, directly though the lumen 28 if necessary, as detailed below. The thickness and permeability of the balloon wall 24 can be modified during its production process. Even in the empty configuration (Fig. 1a), the material of the wall of the balloon is somewhat stretched so that the ball of the balloon is tightly applied to the surface of the inner core 22.

The core 22 contains a magnetic material 23 and a valve26. The valve 26 is designed for piercing by a needle or a needle-like tip at the end of an inserting device, in order to introduce into the balloon of the device 20a solution or suspension. The valve 26 may be a self-sealing valve, any one-way valve, such as a duckbill valve or a ball valve. The magnetic material 23 includes a slot 23x through which the solution or suspension passes before entering between the wall 24 of the balloon and the surface of the core 22 to inflate and form the balloon 8.The solution or suspension enters between the wall of the balloon 24 and the core 22 causing the material of the wall of the balloon to expand until the balloon acquires its desired configuration (Fig. 1B).

Since the wall of the balloon 24 is initially stretched in the empty configuration of the device, the wall of the balloon will be stretched regardless of the amount of fluid introduced into the balloon. Thus, a given device may be used to contain a selectable amount of fluid over a range of volumes. The elasticity of the wall of the balloon allows the balloon to spontaneously contract as fluid leaves the lumen 28 of the balloon.

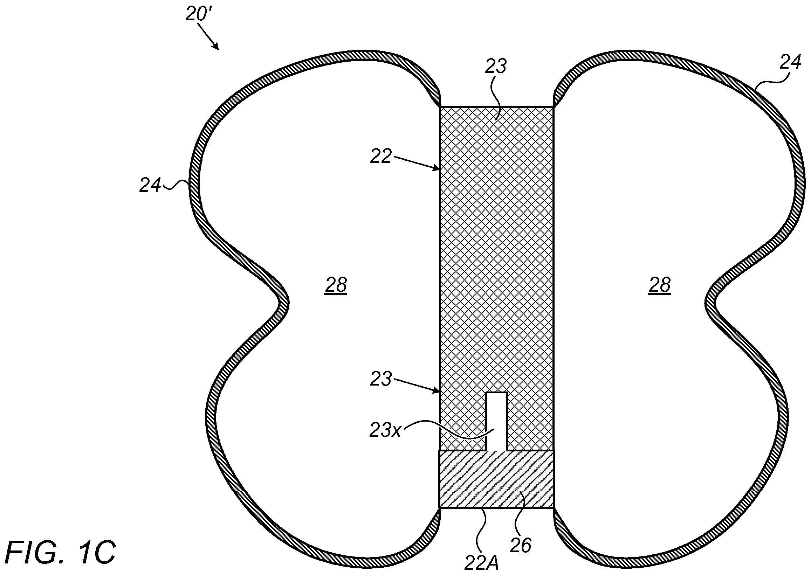

The device can expand to, a spherical shape (as shown in Fig. 1B) or a substantially spherical shape. FIG. 1C shows a device 20' of the invention in which the inflated balloon has a *"dimpled surface",* in order to increase the surface area of the balloon and enhance the rate of release of the drugs.

As stated above, the balloon wall 24 is made from an elastic material that allows drugs to permeate or diffuse through the wall 24. In the case of the urinary bladder, drugs diffusing though the wall 24 are released into the urine and contact the urothelium of the bladder, so that the drug is locally administered.

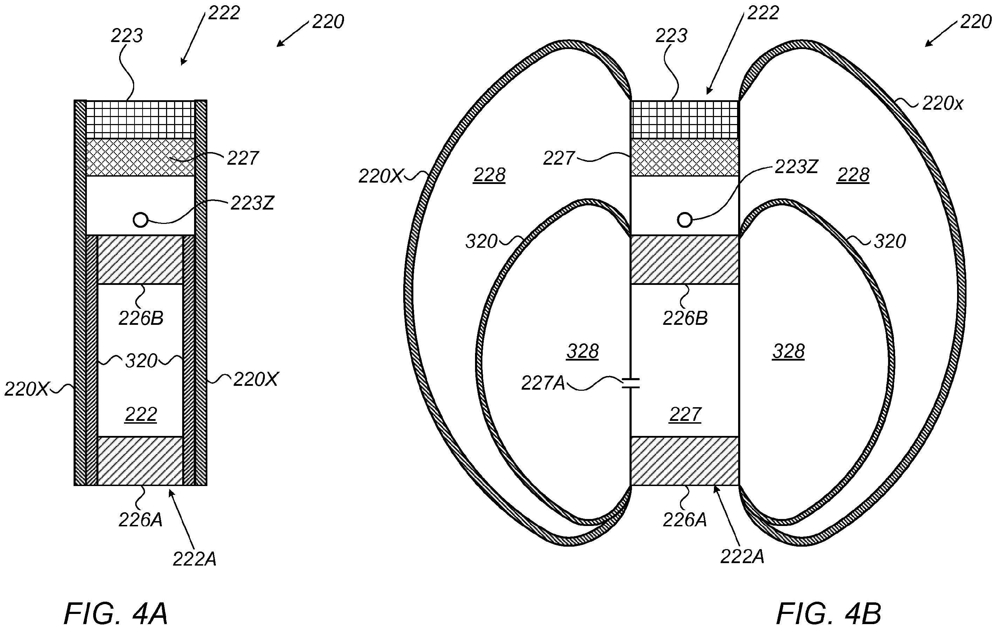

The shape of the expanded balloon can be spherical (Fig. 1b), ovoid, flattened or multifaceted. The inner cavity or lumen 28 of the balloon can be divided into two or more cavities or lumens. The balloon can also be formed of separate 28, 28' lumens, or otherwise segregated lumens, as shown, for example, by the balloon devices220, 220' of FIGS. 4A and 4B, respectively.

The balloon can be filled with oil and one or more oil soluble drugs. The oil provides buoyancy as well as a drug carrying matrix. Alternately, the balloon can be inflated with a combination of oil and water/saline, the water/saline containing one or more water soluble drugs. The oil part is for buoyancy but can also contain oil soluble drugs. The proportions of oil and water depend on the weight of the balloon, enough to give the balloon sufficient buoyancy in urine.

The balloon may have a positive buoyancy for allowing floating (its weight less than the buoyant force) or a neutral buoyancy for allowing "hovering" (its weight equal to the buoyant force). In the case of the urinary bladder, buoyancy of the balloon devices 2 reduces contact between the balloon and the trigone region of the bladder which is rich in sensory nerve ends and is situated near the base of the bladder. Should the balloon device have negative buoyancy, it will sink to the bottom of the bladder, contacting the trigone region possibly causing irritation and pain.

The device is such that during normal use, it may contact the bladder inner surface. At the end of urination, a part of the device may contact the trigone region. Buoyancy of the device allows the device to float on the urine as urine accumulates in the bladder, reducing further irritation.

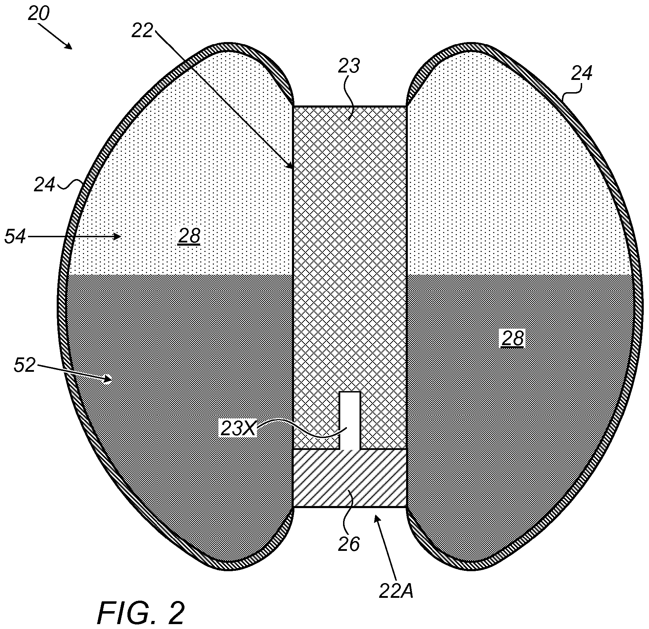

Attention is now directed to FIGS. 2A and 2B, which show balloon devices 20, 20' with filled lumens 28, for example, with one or more drug solutions, buoyancy agents, gases and the like. The filling has expanded the balloon device 20, and along with balloon

Attention is now directed to FIG. 2, which shows the device20after being inflated with a combination of an aqueous solution or suspension of one or more drugs and an oil-based solution or suspension of one or more drugs. The aqueous solution 54 separates from the oil based solution 52 in the lumen 28. Two different drugs may be delivered from the balloon device 20, one from the water based solution 54 and one from the oil based solution 52, with the oil based solution also providing buoyancy to the device 20. In other embodiments, gases, such as air, can be also be inserted into the lumen 28. These gases provide buoyancy to the device20.

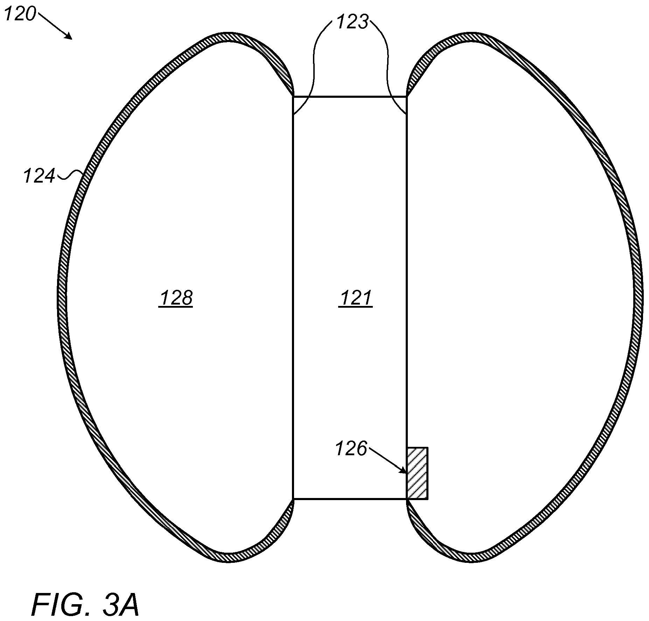

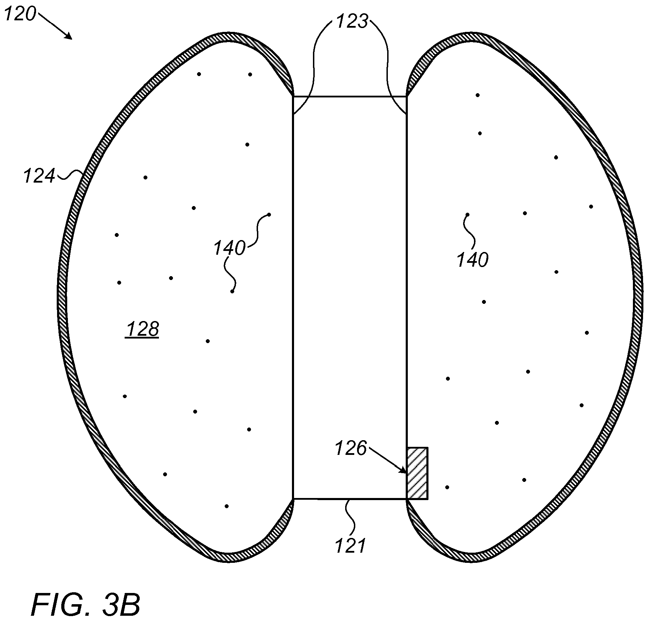

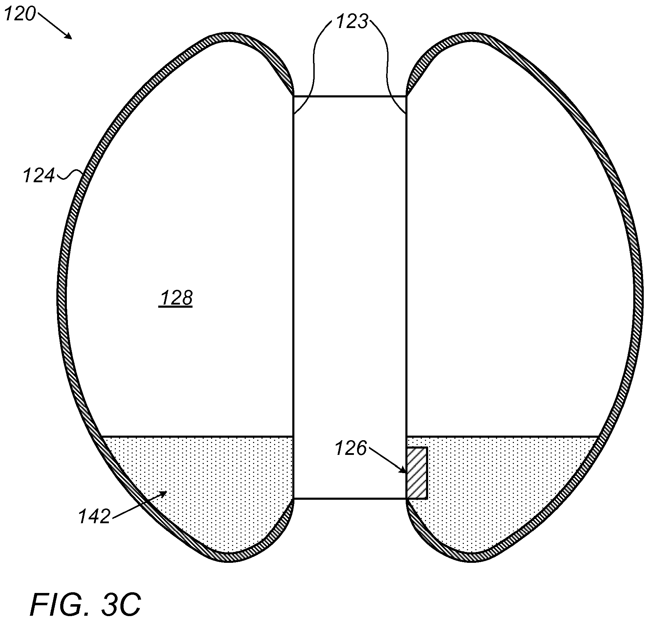

FIG. 3A shows an alternative balloon device 120, in accordance with another embodiment of the invention comprising central hollow cylindrical core 123 made from a rigid or semi-rigid material that may be a permeable material. The core 123 surrounds a channel 121passing through the device 120, and a valve 126, similar to valve 26 detailed above for the device 20that is positioned in the wall of the core 123. All other components of the balloon device 120 are the same or similar to those for the balloon device 20, as described above, and accordingly, have the same reference numerals increased by "100". The device 120may not contain a magnetic element, in which case it can be removed from the bladder using endoscopic grasping forceps. Alternatively, the magnetic elements for the device 120 may be, for example, incorporated or embedded in the wall 124 of the balloon 120 as ferromagnetic particles 140 or strips, as shown in FIG. 3B. These particles 140 are typically added (impregnated/embedded into the wall 124) during balloon production. As another alternative, ferromagnetic particles 142 can be injected into the lumen 128 (dry or in solution), for example, as shown in FIG. 3C.

FIGs. 4A and 4B show a balloon device 220 in accordance with another embodiment of the invention. The balloon device is formed of an outer balloon 220x whose interior includes a separate balloon 320, within the lumen 228 of the outer balloon 220x,. In the device 22, components which are the same or similar to those for the device 20', as described above, and as shown, and accordingly, have the same element numbers increased by "200", for the outer balloon 220x, and increased by "300" for the inner balloon 320. The device 220 has a core 222.

The device 220 is shown in Figs. 4A with B with both balloons 220x and 320 in non-inflated states in an empty configuration, and in Fig. 4B in an inflated configuration. The walls of the two balloons are made from an elastic material that is somewhat stretched prior to filling of the balloons, so that the balloons are tightly applied to the surface of the core 222 in the empty or non-inflated state (Fig. 4A).

After inflation (Fig. 4B), an inner lumen 328 is separate from the outer lumen 228. The core 222 includes two valves 226a, 226b (e.g., self-sealing valves similar to valves 26 detailed above) at the ends of the core 222. The first valve 226a is at an end 222a of the core 222. The core 222 includes openings 227a into the inner lumen 328 of the inner balloon, as accessed through the valve 226a. Materials (substances), such as liquids, oil, water, and gases, are injected into the lumen 328 (via the openings 227a of the tube 227) through the valve 226a to expand it. Through a second valve 226b and an opening 223z in the core 222, materials, such as liquids, such as oil and water, and gases, such as air, are injected into the lumen 228 of the balloon 220x to expand it. The core 222 may include magnetic elements 223, as explained above with reference to the device 20 shown in Fig. 1. For example, the dual lumens 228, 328, may be such that one lumen 228 serves asa reservoir for a water based solution with drugs, and the lumen 328 serves asa reservoir for the oil based solution, without drugs, the oil-based solution providing buoyancy to the balloon device 220. However, the components of the lumens 228, 328 may be switched, if desired. Gases, such as air, may also be added in the amounts desired, into one or both lumens 228, 328, for buoyancy of the balloons 220, 220'.

For example, both balloons 220x, 320 can be inflated separately by using the same needle inserted first through the first valve 226a for filling the inner lumen 328, and then moving through the second valve, to inflate the outer lumen 228. For example, the needle, through which the material is inserted into each lumen 228, 328, can be the same, but the syringe attached to the needle is changed to inject different materials to each separate lumen 328, 228.

The device of the invention can be inserted into the bladder and removed from the bladder, for example by the instrument disclosed in US Patent Application Publication No. US 2010/0016834 A1.

These instruments are advantageous as they do not require any endoscopic manipulation in their operation. Collapse of the balloon 24 will be necessary for removal of the balloon. For this purpose the balloon wall 24 may be punctured with a retrieval instrument, and the residual fluid in the balloon emptied prior to removal of the balloon from the bladder.

Additionally, intravesical drug delivery can be improved by prolonging the total drug exposure and by enhancing the delivery of agents to bladder tissues by enhancing bladder mucosa permeability by intravesical hyperthermia. Also, the addition of a permeability enhancing chemical such as dimethyl sulfoxide (DMSO) into the drug carrying matrix may promote the penetration of water-soluble or lipophilic drugs.

The device may include an externally commanded heating element for increasing the urine temperature to 40-45°C for increasing the urothelial permeability to the drugs.

The device may include a pressure sensor for detecting bladder pressures. When the bladder pressure reaches a predetermined threshold, a command or signal may be given to the heating element to start heating, to increase the urine temperature, as detailed above for increasing the urothelial permeability.

While the balloon has been shown and described above for use in the bladder, the balloon 20 can be used for other intracavitary or regional treatments such as intra-gastric, intra-peritoneal and intra-thecal applications, without or with slight modifications.

### EXAMPLE 1

Experiments were performed using Lidocaine dissolved in oily substances using two different expansion volumes (one with 10ml, the other with 16ml) of the balloon of the device 20. The results showed that a hydrophobic/lipophilic drug, Lidocaine, dissolved in oily substances was released in large quantities through the silicone balloon wall.

A. The balloon was expanded by injection of 10 ml of the oily solution into the balloon.

A series of 4 experiments using lidocaine dissolved in oil was performed. The results were as follows:
1. After 28 days - 436 mg out of 500 mg was released.
2. After 29 days - all the 500 mg was released (out of 500 mg).
3. After 22 days - all the 500 mg was released (out of 500 mg).
4. After 63 days - all the 1000 mg was released (out of 1000 mg).

B. The balloon was expanded by injection of 16 ml of the oily solution into the balloon. 2 groups and a series of 3 experiments using lidocaine dissolved in oil were performed. The results were as follows:
1. After 21 days: - all the 500 mg was released (out of 500 mg)
   - 804 g was released , out of 1000 mg
   - 1086 mg was released, out of 1500 g
2. After 84 days - all the 500 mg was released (out of 500 mg)
   - all the 1000 mg was released (out of 1000 mg)
   - all the 1500 mg was released (out of 1500mg)

The present invention, while shown for human use, is also suitable for animal use.Additionally, while the balloons are shown and described for use in the bladder, this is exemplary, as the balloons and their methods of use, as per the invention, may be used in all areas of the human or animal body.

The descriptions of the various embodiments of the present invention have been presented for purposes of illustration, but are not intended to be exhaustive or limited to the embodiments disclosed. Many modifications and variations will be apparent to those of ordinary skill in the art without departing from the scope of the described embodiments. The terminology used herein was chosen to best explain the principles of the embodiments, the practical application or technical improvement over technologies found in the marketplace, or to enable others of ordinary skill in the art to understand the embodiments disclosed herein.

The terms *"comprises", "comprising", "includes", "including", "having"* and their conjugates mean *"including but not limited to".*

As used herein, the singular form *"a", "an"* and *"the"* include plural references unless the context clearly dictates otherwise. For example, the term *"a compound"* or *"at least one compound"* may include a plurality of compounds, including mixtures thereof.

The word *"optionally"* is used herein to mean *"is provided in some embodiments and not provided in other embodiments".* Any particular embodiment of the invention may include a plurality of *"optional"* features unless such features conflict.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

## Claims

1. A medical device (20) for insertion into a body cavity comprising:
• a core (22);
• an expandable balloon surrounding the core (22), the balloon having a wall (24) made from an elastic permeable material,; and
• a valve (26) configured to allow introduction of one or more fluids between the wall of the balloon and the surface of the core;
**characterized in that**
the balloon having an empty configuration and an inflated configuration, the balloon wall (24) being stretched in the empty configuration and applied to a surface of the core (22), and the balloon having a lumen in the inflated configuration.

2. The device according to claim 1 wherein the core (22) is made from a rigid or semi-rigid material.

3. The device according to any one of the previous claims wherein the wall (24) of the balloon forms a fluid impervious seal with the core (22).

4. The device of any one of the previous claims further comprising one or more magnetic elements (23).

5. The device of claim 4 wherein the magnetic elements 823) are embedded in the wall (24) of the balloon.

6. The device according to any one of the previous claims wherein the balloon further comprises one or more additional lumens (28, 28').

7. The device according to any one of the previous claims adapted for insertion into a urinary bladder.

8. The device according to any one of the previous claims wherein the core (22) is hollow.

9. A system comprising:
(a) the implantable medical device (20) according to claim 1:
(b) one or more instruments selected from an insertion instrument for inserting the device into the body cavity and a retrieval instrument for retrieving the device from the body cavity.

## Patentansprüche

1. Eine medizinische Vorrichtung (20) zum Einführen in eine Körperhöhle, umfassend
• einen Kern (22);
• einen expandierbaren Ballon, der den Kern (22) umgibt, wobei der Ballon eine Wand (24) aus einem elastischen, permeablen Material aufweist, und
• ein Ventil (26), das konfiguriert ist, um die Einführung eines oder mehrerer Fluide zwischen der Wand des Ballons und der Oberfläche des Kerns zu ermöglichen;
**dadurch gekennzeichnet, dass**
der Ballon eine entleerte Konfiguration und eine inflatierte Konfiguration aufweist, wobei die Ballonwand (24) in der entleerten Konfiguration gedehnt und an eine Oberfläche des Kerns (22) angelegt ist, und der Ballon in der inflatierten Konfiguration ein Lumen aufweist.

2. Die Vorrichtung gemäß Anspruch 1, wobei der Kern (22) aus einem starren oder halbstarren Material hergestellt ist.

3. Die Vorrichtung gemäß einem der vorhergehenden Ansprüche, wobei die Wand (24) des Ballons eine flüssigkeitsundurchlässige Dichtung mit dem Kern (22) bildet.

4. Die Vorrichtung nach einem der vorhergehenden Ansprüche weiter umfassend ein oder mehrere magnetische Elemente (23).

5. Die Vorrichtung nach Anspruch 4, wobei die magnetischen Elemente (823) in die Wand (24) des Ballons eingebettet sind.

6. Die Vorrichtung gemäß einem der vorhergehenden Ansprüche, wobei der Ballon weiter ein oder mehrere zusätzliche Lumen (28, 28') umfasst.

7. Die Vorrichtung gemäß einem der vorhergehenden Ansprüche, angepasst zum Einführen in eine Harnblase.

8. Die Vorrichtung gemäß einem der vorhergehenden Ansprüche, wobei der Kern (22) hohl ist.

9. Ein System umfassend:
(a) die implantierbare medizinische Vorrichtung (20) gemäß Anspruch 1:
(b) ein oder mehrere Instrumente ausgewählt aus einem Einführungsinstrument zum Einführen der Vorrichtung in die Körperhöhle und einem Entnahmeinstrument zum Entnehmen der Vorrichtung aus der Körperhöhle.

## Revendications

1. Un dispositif médical (20) destiné à être introduit dans une cavité corporelle comprenant :
• un corps (22) ;
• un ballonnet extensible entourant le corps (22), le ballonnet présente une paroi (24) fabriquée à partir de matière élastique perméable ; et
• une vanne (26) conçue pour permettre l'introduction d'un ou de plusieurs fluides entre la paroi du ballonnet et la surface du corps ;
**caractérisé par le fait que**
le ballonnet présente une configuration vide et une configuration gonflée, la paroi (24) du ballonnet étant étirée dans la configuration vide et appliquée à une surface du corps (22), et le ballonnet présentant une lumière dans la configuration gonflée.

2. Le dispositif conforme à la revendication 1, où le corps (22) est conçu à partir d'un matériau rigide ou semi-rigide.

3. Le dispositif conforme à l'une des revendications précédentes, où la paroi (24) du ballonnet forme une membrane étanche avec le corps (22).

4. Le dispositif conforme à l'une des revendications précédentes, comprenant également au moins un élément magnétique (23).

5. Le dispositif conforme à la revendication 4, où les éléments magnétiques (23) sont intégrés à la paroi (24) du ballonnet.

6. Le dispositif conforme à l'une des revendications précédentes, où le ballonnet comprend également au moins une lumière additionnelle (28, 28').

7. Le dispositif conforme à l'une des revendications ci-dessus, adapté à l'introduction dans une vessie.

8. Le dispositif conforme à l'une des revendications ci-dessus, où le corps (22) est creux.

9. Un système comprenant :
(a) le dispositif médical implantable (20) conformément à la revendication 1 ;
(b) un ou plusieurs instruments sélectionnés parmi un outil d'introduction pour l'insertion du dispositif dans la cavité corporelle et un outil d'extraction permettant d'extraire le dispositif de la cavité corporelle.
